(19) Europäisches Patentamt
European Patent Office
Office européen des brevets

(11) Veröffentlichungsnummer: **0 217 018 B1**

(12) **EUROPÄISCHE PATENTSCHRIFT**

(45) Veröffentlichungstag der Patentschrift: **06.03.91**

(51) Int. Cl.[5]: **C07C 227/00**, C07C 229/32

(21) Anmeldenummer: **86109527.1**

(22) Anmeldetag: **11.07.86**

# (54) Verfahren zur Herstellung von 3-Aminoacrylsäureestern.

(30) Priorität: **30.08.85 DE 3531067**

(43) Veröffentlichungstag der Anmeldung:
**08.04.87 Patentblatt 87/15**

(45) Bekanntmachung des Hinweises auf die Patenterteilung:
**06.03.91 Patentblatt 91/10**

(84) Benannte Vertragsstaaten:
**CH DE FR GB LI**

(56) Entgegenhaltungen:
**BE-A- 869 021**
**DE-A- 2 938 872**
**DE-A- 3 037 086**

(73) Patentinhaber: **HÜLS AKTIENGESELLSCHAFT**
**Paul-Baumann-Strasse 1**
**W-4370 Marl 1(DE)**

(72) Erfinder: **Engländer, Fritz, Dr.**
**Flossweg 10**
**W-5300 Bonn 2(DE)**
Erfinder: **El-Chahawi, Moustafa, Dr.**
**Pacellistrasse 4**
**W-5210 Troisdorf(DE)**
Erfinder: **Vogt, Wilhelm, Dr.**
**Kleiststrasse 39**
**W-5000 Köln 40(DE)**

EP 0 217 018 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein Verfahren zur Herstellung von 3-Aminoacrylsäureestern.

Die Herstellung von N,N-Dimethylaminoacrylsäureethylestern durch Umsetzung von Natrium-3-hydroxyacrylsäureethylestern mit Dimethylamin HCl in absolutem Ethanol (Anales de Chimie 10, 18 (1932)108) ist bekannt.

Die Synthese hat jedoch mehrere Nachteile:
Die Resktionszeit ist mit 7 bis 8 Stunden sehr lang. Trotzdem wird nur eine Ausbeute von 41% erreicht. Die Aufarbeitung des Reaktionsgemisches nach beendeter Umsetzung zur Abtrennung des entatandenen NaCl ist technisch sehr aufwendig.

Bekannt war bereits aus DE-A-30 37 086, m-Chloranilin, ein aromatisches Amin, mit Alkali-3-hydroxiacrylsäureester umzusetzen, wobei die Umsetzung in einem Lösungsmittelgemisch aus Wasser und einem Alkohol erfolgt und betont wird, daß die Anwesenheit von Wasser und Alkohol für das Entstehen des dortigen Zielprodukts und für die Ausbeute wesentlich ist. In dieser DE-A wird auch Alkalihydroxyacrylsäureester als Feststoff oder suspendiert bzw. gelöst in Wasser genannt.

Es wurde nun gefunden, daß 3-Aminoacrylsäureester der allgemeinen Formel (I) in ausgezeichneten Ausbeuten und hohen Reinheiten erhalten werden, wenn die Umsetzung von Alkali-3-hydroxyacrylsäureestern der allgemeinen Formel (II) mit den Salzen der Amine der allgemeinen Formel (III) gemäß Patentanspruch durchgeführt wird.
Sowohl die Aminsalze als auch die Alkali-3-hydroxyacrylsäureester sind in wasser leicht löslich. Wegen der Instabilität von Alkali-3-hydroxyacrylsäureethylester in wässriger Lösung empfiehlt es ich, bei der Durchführung der Reaktion den Alkali-3-hydroxyacrylsäureethylestern zu der wässrigen Lösung des Aminsalzes zu dosieren. Überraschend läuft die Reaktion praktisch momentan ab und der gebildete Aminoacrylsäureester trennt sich von der Wasserphase.

Gegenstand der Erfindung ist das Verfahren nach Anspruch 1, wonach die Stoffe der Formel (I) aus den Ausgangsstoffen (II) und (III), welche Stoffe gemäß patentanspruch definiert sind, durch Umsetzung in wässrigen Lösungen der Aminsalze der Formel (III) durch Zugabe der Alkalihydroxyacrylsäureester der Formel (II) als fester Stoff oder suspendiert in Methylformiat, Ethylformiat, Methylacetat, Ethylacetat, Toluol, Dimethyloxyethan oder Acetonitril ausgeführt werden. Vorzugsweise werden die Alkali-3-hydroxyacrylsäureester der Formel (II) in den im Anspruch 1 genannten Lösungsmitteln, insbesondere in Ethylacetat, als bei der Herstellung von (II) entstandene Suspension zugegeben.

Die Zugabe des Natrium-3-hydroxyacrylsäureesters zur wässrigen Aminsalzlösung kann sowohl in Form der festen Verbindung als auch suspendiert in Methylformiat, Ethylformiat, Methylacetat, Ethylacetat, Toluol, Dimethyloxyethan oder Acetonitril erfolgen. Als suspensionsmittel sind vorzugsweise solche geeignet, die nicht in jedem Verhältnis mit Wasser mischbar sind und auch schon bei der Herstellung des Alkali-3-hydroxyacrylsäureesters zugegen sein können.

Die hier bevorzugte Herstellung von Alkali-3-hydroxyacrylsäureethylester besteht in der Einwirkung von CO auf eine Suspension von Na- oder K-Ethylat oder -methylat in Ethylacetat, so daß nach beendeter Reaktion Natrium-3-hydroxyacrylsäureethylester in überschüssigem Ethylacetat suspendiert ist. Diese Suspension kann di rekt mit der wässrigen Lösung des Aminsalzes umgesetzt werden. Es ist auch möglich, das Alkali-Salz des 3-Hydroryacrylsäureethylesters abzufiltrieren und ethylacetatfeucht oder auch getrocknet umzusetzen.

Ausbeuten von mehr als 75 %, bezogen auf das eingesetzte Alkali-Salz, sind erreichbar. Trimesinsäureester wird überraschenderweise nicht gebildet. Der Aminoacrylsäureester, der sich aus der Wasserphase abscheidet, soweit nicht zusätzlich organische Lösungsmittel anwesend sind, ist bereits sehr rein. Nach einer Flash-Destillation, die hauptsächlich zur Abtrennung geringer anorganischer Anteile durchgeführt wird, erhält man ein Destillat mit einer durchschnittlichen Reinheit von 99,5%.

Die Umsetzung von Alkali-3-hydroxyacrylsäureester mit Aminsalzen findet bei 20 bis 50° C, vorzugsweise bei 20 bis 30° C statt. Niedrigere Temperaturen bringen keine Vorteile, höhere Temperaturen als 50° C führen zu geringeren Ausbeuten.

Das Aminsalz wird vorteilhafterweise im Überschuß angewendet, und zwar kommt auf 1 Mol Alkali-3-hydroxyacrylsäureester 1,05 bis 2,5 Mol Aminsalz zum Einsatz, wobei der Bereich 1,1 bis 1,2 Mol bevorzugt ist, so daß gemäß Anspruch 3 die Aminsalze der Formel (III) in einem Überschuß von 0,05 bis 1,5 Mol angewandt werden. Die Art des Anions des Aminsalzes ist für die Umsetzung im allgemeinen nicht Kritisch. Es Können sowohl Chloride als auch Sulfate verwendet werden. Darüber hinaus ist auch der Einsatz organischer Säuren möglich. Vorteilhafterweise wird das Salz in gelöster Form in Wasser zur Reaktion eingesetzt. Als Alkalisalz in den Estern der Formel (II) ist Na bevorzugt und K möglich.

Die Amine der Aminsalze sind primäre bzw. sekundäre Amine oder Ammoniak. Tertiäre Amine

reagieren nicht.

In den Produkten und Ausgangsstoffen sind als $R_1$ oder $R_2$ Alkyl oder H sehr bevorzugt. Dialkylamine sind bevorzugt gegenüber Monoalkylaminen. In Dialkylaminen sind vorzugsweise $R_1$ und $R_2$ gleich und haben bevorzugt 1 bis 4 C-Atome.

Weiterhin sind bevorzugt Piperidin und ggf. Methylpiperidine. Als $R_4$ sind Alkylreste mit 1 bis 4 C-Atomen, besonders Methyl und Ethyl bevorzugt.

Als $R_3$ sind Alkylreste mit 1 bis 4 C-Atomen, besonders Methyl und Ethyl, sowie Benzyl und Phenyl bevorzugt.

Die Wassermenge bei der Umsetzung wird vorzugsweise so gewählt daß nach beendeter Reaktion eine 20 bis 25 Gew.%ige NaCl-Lösung resultiert, wodurch die Abtrennung des Reaktionsproduktes erleichtert wird.

Die Aminoacrylsäureester sind Zwischenprodukte zur Synthese heterocyclischer Verbindungen, beispielsweise zur Synthese von 4-Hydroxychinoline (Angew. Chem. 50 (1947); J.Am.Chem.Soc. 68 (1946)-1256).

## Beispiel 1

138 Gew.-Teile festes getrocknetes Natrium-3-hydroxyethylacrylat wird in eine Lösung von 90 Gew.-Teilen Dimethylamin . HCl in 250 Gew.-Teile Wasser gegeben. Nach kurzer Zeit scheidet sich ein gelbes Öl an. Das Öl wird abgetrennt und die Wasserphase mit Diethylether extrahiert. Bei der sich anschließenden Vakuumdestillation erhält man 107,3 Gew.-Teile N,N-Dimethylaminoacrylsäureethylester mit einer durch Gaschromatographie ermittelten Reinheit von 99,7% , entsprechend einer Ausbeute vor 74,8%.

## Beispiel 2

In 340 Gew.-Teile Ethylacetat werden 68 Gew.-Teile Natriumethylat suspendiert, 5 Gew.-Teile Ethylformiat zugesetzt und das Gemisch bei 50° C und einem CO-Druck von 50 bar gerührt. Nach 2 Stunden wird kein CO mehr aufgenommen. Das Reaktionsgemisch wird auf 25° C abgekühlt und in eine Lösung von 90 Gew.-Teilen Dimethylamin . HCl in 250 Gew.-Teile Wasser einlaufen gelassen, so daß eine Temperatur von 30° C nicht überschritten wird. Es wird noch zwei Stunden nachgerührt und dann die organische von der wässrigen Phase abgetrennt. Nach dem Abdestillieren des Ethylacetats erhält man bei der an sich anschließenden Vakuumdestillation 103,5 Gew.-Teile N,N-Dimethylaminoacrylsäureethylester mit einer Reinheit von 98 %, was einer Ausbeute von 70,2 % bezogen auf eingesetztes Natriumethylat entspricht.

## Beispiel 3

50,6 Gew.-Teile Methylaminhydrochlorid werden in 100 Gew.-Teilen Wasser gelöst und 69 Gew.-Teilen getrocknetes Natrium-3-hydroxyethylacrylat zugegeben. Die Mischung wird 2 Stunden gerührt und mit Ether extrahiert. Nach Destillation erhält man 41,0 Gew.-Teile Methylaminoacrylsäureethylester, entsprechend einer Ausbeute von 63,6 % d. Th.

## Beispiel 4

61,2 Gew.-Teile Ethylaminhydrochlorid werden in 100 g Gew.-Teilen Wasser gelöst und 69 Gew.-Teile getrocknetes Natrium-3-hydroxyethylacrylat zugegeben. Die Aufarbeitung erfolgt analog Beispiel 3. Ausbeute: 54 Gew.-Teile Ethylaminoacrylsäureethylester, entsprechend einer Ausbeute von 75,5 % d. Th.

## Beispiel 5

Zu einer Lösung von 50 Gew.-Teilen $NH_4Cl$ in 200 Gew.-Teilen Wasser werden 69 g Natrium-3-hydroxyethylacrylat gegeben und 2 Stunden gerührt. Die Mischung wird mit Ether extrahiert und der Ether im Vakuum abdestilliert. Man erhält 51 g Rückstand mit einem Anteil von 21% Aminoacrylsäureethylester.

Beispiel 6

102 Gew.-Teile Piperidin werden mit 175 Gew.-Teilen Wasser versetzt und 119 g konzentrierte Salzsäure zugegeben. Dann wird die Lösung bei Raumtemperatur mit 138 Gew.-Teilen Natrium-3-hydroxyacrylsäureethylester versetzt, 2 Stunden gerührt und mit Ethylacetat extrahiert. Bei der destillativen Aufarbeitung erhält man 123 Gew.-Teile 3-Piperidinoacrylsäureethylester entsprechend einer Ausbeute von 67,2% d. Th.

Beispiel 7

87,6 Gew.-Teile n-Butylamin, 177 Gew.-Teile Wasser und 117 Gew.-Teile konzentrierte Salzsäure werden mit 138 Gew.-Teilen Natrium 3-hydroxyacrylsäureethylester versetzt, 2 Stunden gerührt und mit Diethylether extrahiert. Bei der destillativen Aufarbeitung erhält man 104 Gew.-Teile 3-(n-Butylamino)-acrylsäureethylester entsprechend einer Ausbeute von 60,8% d. Th.

Beispiel 8

97 Gew.-Teile Dimethylamin.HCl werden in 250 Gew.-Teilen Wasser gelöst und mit 200 Gew.-Teilen Natrium-3-hydroxy-2-phenyl-acrylsäuremethylester versetzt. Die Mischung wird 2 Stunden gerührt, die organische Phase abgetrennt und destilliert. Man erhält 148 Gew.-Teile 3-Dimethylamino-2-phenyl-acrylsäuremethylester entsprechend einer Ausbeute von 72.2 d. Th.

**Ansprüche**

1. Verfahren zur Herstellung von 3-Aminoacrylsäureestern der allgemeinen Formel

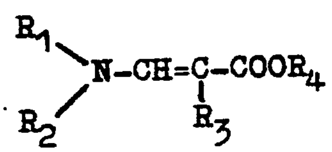

(I)

in der $R_1$ und $R_2$ die Bedeutung H, gleiche oder verschiedene geradkettige oder verzweigte Alkylreste mit 1 bis 8 C-Atomen Alkenyl-Reste mit 1 bis 8 C-Atomen, $R_3$ die Bedeutung H, geradkettige oder verneigte Alkylreste mit 1 bis 8 C-Atomen, isocyclischer oder heterocyclischer oder aromatischer Ring oder in der $R_1$ und $R_2$ gemeinsam Bestandteil eines den Aminstickstoff oder zusätzlich ein weiteres Stickstoffatom enthaltenden 5- bis 7-gliedrigen Ringes mit einer oder zwei oder ohne Doppelbindungen, $R_4$ geradkettige oder verzweigte Alkylreste mit 1 bis 8 C-Atomen, isocyclischer oder aromatischer Ring haben, durch Umsetzung von Alkalisalzen von ß-Hydroxyacrylsäureestern der allgemeinen Formel

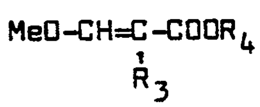

(II)

in der $R_3$ und $R_4$ die obenstehende Bedeutung haben und Me ein Alkalimetall ist mit Aminsalzen der allgemeinen Formel

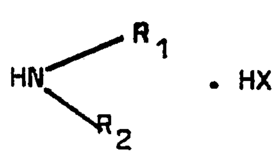

(III),

in der $R_1$ und $R_2$ die obenstehende Bedeutung haben und X ein Anion einer anorganischen oder organischen Säure ist, **dadurch gekennzeichnet,** daß die Umsetzung in wässrigen Lösungen der Aminsalze der Formel (III) durch Zugabe der Alkalihydroxyacrylsäureester der Formel (II) als fester Stoff oder suspendiert in Methylformiat, Ethylformiat, Methylacetat, Ethylacetat, Toluol, Dimethyloxyethan oder Acetonitril ausgeführt wird.

**2.** verfahren nach Anspruch 1, dadurch gekennzeichnet, daß die Alkalisalze der ß-Hydroxyacrylsäureester der Formel (II) in den im Anspruch 1 genannten Lösungsmitteln, insbesondere in Ethylacetat, als bei der Herstellung von (II) entstandene Suspension zugegeben werden.

**3.** Verfahren nach einem der Ansprüche 1 oder 2, dadurch gekennzeichnet, daß die Aminsalze der Formel (III) in einem Überschuß von 0,05 bis 1,5 Mol angewandt werden.

## Claims

**1.** Process for the production of 3-amino acrylic acid esters of the general formula.

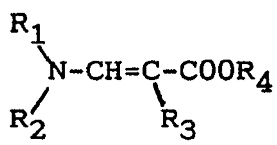

(I)

in which $R_1$ and $R_2$ have the meaning H, the same or different straight chain or branched alkyl residues with 1 to 8 C-atoms, alkenyl residues with 1 to 8 C-atoms, $R_3$ has the meaning H, straight chain or branched alkyl residues with 1 to 8 C-Atoms, isocyclic or heterocyclic or aromatic ring or in which $R_1$ and $R_2$ are together a constituent of a 5 - to 7- membered ring with 1 or 2 or without double bonds and containing the amine nitrogen or additionally a further nitrogen atom, $R_4$ is straight chain or branched alkyl residue with 1 to 8 C-atoms, isocyclic or aromatic ring, by reaction of alkali salts of $\beta$-hydroxyacrylic acid esters of the general formula

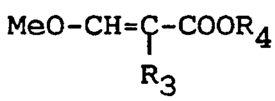

(II)

in which $R_3$ and $R_4$ have the above set out meaning and Me is an alkali metal, with amine salts of the general formula

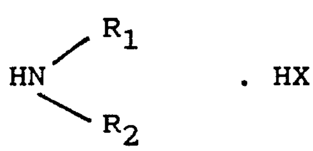

(III)

in which $R_1$ and $R_2$ have the above set out meaning and X is an anion of an inorganic or organic acid, characterised in that the reaction is conducted in aqueous solutions of the amine salt or formula (III) by addition of the alkali hydroxyacrylic acid ester of formula (II) as solid substance or suspended in methyl formate, ethyl formate, methyl acetate, ethyl acetate, toluene, dimethyloxyethane or acetonitrile.

**2.** Process according to claim 1, characterised in that the alkali salts of the $\beta$-hydroxyacrylic acid esters of formula (II) are added to the solvents indicated in claim 1, in particular to ethyl acetate, as suspension produced in the production of (II).

**3.** Process according to 1 of claims 1 or 2, characterised in that the amine salts of formula (III) are used in an excess of 0.05 to 1.5 mol.

## Revendications

1. Procédé de préparation d'esters d'acides 3-amino-acryliques de formule générale :

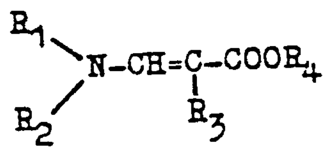

(I)

(dans laquelle $R_1$ et $R_2$ représentent H des restes alkyles identiques ou différents , linéaires ou ramifiés, ayant 1 à 8 atomes de carbone, des restes alcényles ayant 1 à 8 atomes de carbone; $R_3$ représente H , des restes alkyles linéaires ou ramifiés ayant 1 à 8 atomes de carbone, un noyau isocyclique ou hétérocyclique ou aromatique, ou bien $R_1$ et $R_2$ font ensemble partie d'un noyau pentagonal à heptagonal contenant l'azote de la fonction amine ou contenant en outre un autre atome d'azote, et ayant une ou deux ou bien pas de doubles liaisons; $R_4$ représente des restes alkyles linéaires ou ramifiés ayant 1 à 8 atomes de carbone ou un noyau isocyclique ou aromatique ) par réaction de sels alcalins d'esters d'acides $\beta$-hydroxyacryliques de formule générale:

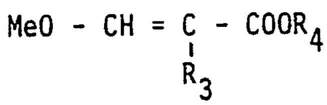

( II)

(dans laquelle $R_3$ et $R_4$ ont le sens précité et Me représente un métal alcalin ), avec des sels d'amines de formule générale :

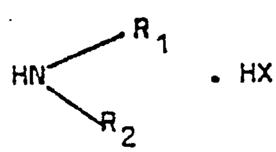

(III),

( dans laquelle $R_1$ et $R_2$ ont le sens précité et X représente un anion d'un acide minéral ou organique ), procédé caractérisé en ce qu'on conduit la réaction dans des solutions aqueuses des sels d'amines de formule (III) par addition des sels alcalins des esters d'acides hydroxyacryliques de formule (II) sous forme de solide ou en suspension dans du formiate de méthyle , du formiate d'éthyle , de l'acétate de méthyle , de l'acétate d'éthyle , du toluène , du diméthyloxyéthane ou de l'acétonitrile .

2. Procédé selon la revendication 1 ,caractérisé en ce qu'on ajoute les sels alcalins des esters d'acides $\beta$-hydroxyacryliques de formule (II) dans les solvants cités à la revendication 1 ,notamment dans de l'acétate d'éthyle, sous forme de la suspension obtenue lors de la prépara tion de (II).

3. Procédé selon l'une des revendications 1 ou 2 , caractérisé en ce qu'on utilise les sels d'amines de formule (III) en un excès de 0,05 à 1,5 mole .